# EUROPEAN PATENT APPLICATION

(11) **EP 4 840 948 A1**
(43) Date of publication of application: **18.11.2026**
(21) Application number: 24916536.6
(22) Date of filing: 30.10.2024
(51) Int. Cl.: C12N 9/10, C12N 15/54, C12N 15/70, C12N 1/21, C12N 11/14

(54) **CURABLE AND REUSABLE HELICOBACTER PYLORI FUCOSYLTRANSFERASE MUTANT**

(30) Priority: 14.01.2024 CN 202410049962
(71) Applicant: Ray-Medicine Inc., Haidian District, Beijing 100094 (CN)
(72) Inventor: WANG, Jishu, Beijing 100094 (CN); HUANG, Tao, Beijing 100094 (CN)
(74) Representative: dompatent
(86) International application number: PCT/CN2024/128670
(87) International publication number: WO 2025/148485

(57) **Abstract**

Disclosed in the present invention is a Helicobacter pylori α-1,3-fucosyltransferase mutant, a polynucleotide encoding the mutant, and an expression vector and host cell containing the polynucleotide. Further disclosed in the present invention is a method for site-specific conjugation of the Helicobacter pylori α-1,3-fucosyltransferase mutant to magnetic beads. According to the method, fucosyltransferase can be repeatedly utilized and can be quickly and effectively removed from an enzymatic reaction system. Compared with a wild type, the Helicobacter pylori α-1,3-fucosyltransferase mutant provided by the present invention has a higher yield, and the fucosyltransferase can be effectively fixed on a solid surface, such as the surface of a magnetic bead, by means of a more preferred method without compromising the activity. By means of a magnetic separation method, the fucosyltransferase is separated from the enzymatic reaction system and washed for reuse.

## Description

### FIELD OF THE INVENTION

The present invention discloses a mutant of fucosyltransferase, belonging to the technical field of enzyme engineering.

### BACKGROUND ART

The α-1,3-fucosyltransferase of Helicobacter pylori is a branched fucosylating enzyme that transfers fucose from GDP-fucose (donor substrate) to GlcNAc (N-acetylglucosamine) and LacNAc (N-acetyllactosamine) on N-glycan branches (receptor substrates). This reaction is a rapid unidirectional enzymatic reaction with high regioselectivity and stereoselectivity. Compared with chemical methods, it has the characteristics of mild conditions and high yield. However, a constraint on industrialization is how to rapidly separate or remove fucosyltransferase after the enzymatic reaction. Although fucosyltransferase can be removed by affinity purification through chromatography columns and membranes, the recovery and reuse of the enzyme also becomes a problem. In industrial production, to ensure high efficiency of enzymatic reactions, excess enzyme is usually added. Reusing enzymes can not only reduce production costs and minimize waste, but also reduce the environmental impact of waste.

CN114369585A discloses a recombinant truncated form of α-1,3-fucosyltransferase from Helicobacter pylori (FucTd), which has good stability and is not consumed or destroyed during the reaction process, meaning they have the potential for reuse. How to continuously utilize or optimize the utilization process has become an urgent problem to be solved in the industrialization process. The object of the present invention is to provide a reusable mutant of Helicobacter pylori α-1,3-fucosyltransferase without reducing enzyme activity.

### TECHNICAL SOLUTIONS

Based on the above objectives, the present invention utilizes PCR mutation principles, based on the recombinant truncated form of Helicobacter pylori alpha-1,3-fucosyltransferase (strain ATCC 700392/26695) (FucTd) (sequence shown as SEQ ID NO.1), to obtain mutants of Helicobacter pylori α-1,3-fucosyltransferase (Met1-Asn405-HIS×6, including from the 1st amino acid (methionine Met) to the 405th amino acid (asparagine Asn) and fused with a purification tag composed of 6 histidines at the C-terminus). The truncated form (FucTd) and mutants were respectively induced and expressed in E. coli, and mutants with soluble expression were selected and purified. Based on the above mutation strategy, the present invention first provides a mutant of Helicobacter pylori α-1,3-fucosyltransferase, wherein: the amino acid sequence of said mutant of Helicobacter pylori α-1,3-fucosyltransferase is shown as SEQ ID NO.3; in the present invention, the mutant having this sequence is relative to the Helicobacter pylori α-1,3-fucosyltransferase having the amino acid sequence shown as SEQ ID NO.1 (named "FucTd" in the present invention), with cysteine to serine mutations at both the 169th and 238th amino acid positions (C169S and C238S); said mutant is named "FucTd-2C" in the present invention; or
The amino acid sequence of said mutant of Helicobacter pylori α-1,3-fucosyltransferase is shown as SEQ ID NO.5; in the present invention, the mutant having this sequence is relative to the Helicobacter pylori α-1,3-fucosyltransferase having the amino acid sequence shown as SEQ ID NO.1, with a cysteine insertion mutation at the 406th amino acid position (406C); said mutant is named "FucTd_C406" in the present invention; or
The amino acid sequence of said mutant of Helicobacter pylori α-1,3-fucosyltransferase is shown as SEQ ID NO.7; in the present invention, the mutant having this sequence is relative to the Helicobacter pylori α-1,3-fucosyltransferase having the amino acid sequence shown as SEQ ID NO.1, with a cysteine insertion mutation at the 412th amino acid position (412C); said mutant is named "FucTd_C412" in the present invention; or
The amino acid sequence of said mutant of Helicobacter pylori α-1,3-fucosyltransferase is shown as SEQ ID NO.9; in the present invention, the mutant having this sequence is relative to the Helicobacter pylori α-1,3-fucosyltransferase having the amino acid sequence shown as SEQ ID NO.1, with cysteine to serine mutations at both the 169th and 238th amino acid positions (C169S and C238S), and a cysteine insertion mutation at the 406th amino acid position (406C); said mutant is named "FucTd-2C_C406" in the present invention.

Secondly, the present invention provides a polynucleotide encoding said mutant of Helicobacter pylori α-1,3-fucosyltransferase. Based on common knowledge in the art and following the triplet codon rules of protein coding, the same amino acid can be encoded by different triplet nucleotides. Therefore, any polynucleotide capable of encoding the mutant of Helicobacter pylori α-1,3-fucosyltransferase having the amino acid sequence shown as SEQ ID NO.3, 5, 7, or 9 falls within the scope of the polynucleotide encoding said mutant of Helicobacter pylori α-1,3-fucosyltransferase as defined in the present invention.

In a preferred technical solution, the polynucleotide sequence encoding the mutant of Helicobacter pylori α-1,3-fucosyltransferase having the amino acid sequence shown as SEQ ID NO.3 is shown as SEQ ID NO.4; or
The polynucleotide sequence encoding the mutant of Helicobacter pylori α-1,3-fucosyltransferase having the amino acid sequence shown as SEQ ID NO.5 is shown as SEQ ID NO.6; or
The polynucleotide sequence encoding the mutant of Helicobacter pylori α-1,3-fucosyltransferase having the amino acid sequence shown as SEQ ID NO.7 is shown as SEQ ID NO.8.

The polynucleotide sequence encoding the mutant of Helicobacter pylori α-1,3-fucosyltransferase having the amino acid sequence shown as SEQ ID NO.9 is shown as SEQ ID NO.10.

Thirdly, the present invention provides an expression vector expressing the above-mentioned mutant of Helicobacter pylori α-1,3-fucosyltransferase, wherein said expression vector contains the above-mentioned polynucleotide. In a specific embodiment of the present invention, said vector is a commercially available MilliporeSigma^{™} pET-41a(+) DNA Vector; other conventional expression vectors in the field of genetic engineering can also be applied in the present invention.

Fourthly, the present invention provides a host cell expressing the above-mentioned mutant of Helicobacter pylori α-1,3-fucosyltransferase, wherein said host cell contains the above-mentioned expression vector. In a specific embodiment of the present invention, said host cell is E. coli BL21(DE3); other conventional host cells in the field of genetic engineering can also be applied in the present invention.

Fifthly, the present invention provides the above-mentioned mutant of Helicobacter pylori α-1,3-fucosyltransferase coupled with a solid phase carrier.

In a preferred embodiment, said solid phase carrier is magnetic beads.

Sixthly, the present invention provides a method for preparing the above-mentioned mutant of Helicobacter pylori α-1,3-fucosyltransferase coupled with magnetic beads, said method comprising the following steps:
(1) Directly coupling free thiol groups on the mutant of fucosyltransferase to the surface of magnetic beads, or
(2) Performing site-specific biotinylation of free thiol groups, and then coupling to magnetic beads by means of biotin-streptavidin affinity.

Finally, the present invention provides a method for repeatedly applying the above-mentioned mutant of Helicobacter pylori α-1,3-fucosyltransferase coupled with a solid phase carrier to transfer a donor substrate coupled with a biomacromolecule to a receptor substrate, said method comprising the following steps:

After transferring GDP-fucose coupled with a biomacromolecule (donor substrate) to target cells (receptor substrate), washing the magnetic beads in a magnetic field environment, removing the supernatant, adding fresh donor substrate and receptor substrate, and again transferring the donor substrate coupled with a biomacromolecule to the receptor substrate.

In a specific embodiment of the present invention, said donor substrate is GDP-fucose, and said receptor substrate is target cells; in a more specific embodiment, said target cells are NK-92 cells; in another specific embodiment of the present invention, the number of repetitions is 5.

In a preferred embodiment, said biomacromolecule is an antibody. In a specific embodiment of the present invention, said antibody is trastuzumab.

### BENEFICIAL EFFECT

After analysis on mutating and expressing the amino acid sequence of the recombinant truncated form of Helicobacter pylori α-1,3-fucosyltransferase (Met1-Asn405-HIS×6, FucTd), the present invention found that fucosyltransferase has only two cysteines at positions 169 and 238, but these two cysteines do not participate in the protein spatial structure of the enzyme through disulfide bond formation. After mutating them to other amino acids, such as serine (Ser), there were no significant changes in expression level, solubility, and enzyme activity in E. coli.

Furthermore, retaining the two cysteines at positions 169 and 238 of the recombinant truncated form of fucosyltransferase (FucTd) and adding additional cysteines at the end of the amino acid sequence, there were no significant changes in solubility and enzyme activity, and no aggregate formation was observed. The overall expression level and yield instead increased by 3-5 times. This indicates that the two cysteines at positions 169 and 238 are not exposed on the protein molecule surface, and the added free cysteines have no effect on yield and enzyme activity. Experiments with maleimide (NHS) for highly selective site-specific biotinylation to free thiol groups on proteins under mild conditions (as in Example 5) also confirmed that the two cysteines at positions 169 and 238 cannot form adducts with NHS, further demonstrating that the two cysteines at positions 169 and 238 are buried inside the protein molecule structure. Based on these findings, the present invention utilizes site-specific coupling technology of free cysteines to couple mutants of Helicobacter pylori α-1,3-fucosyltransferase (such as FucTd_C406, FucTd_C412, FucTd-2C_C406) to the surface of magnetic beads. Compared with free enzyme, the activity of fucosyltransferase coupled to magnetic beads was not only unaffected but also improved compared to the catalytic activity of free enzyme. After the catalytic reaction of fucosyltransferase coupled to magnetic beads is completed, the fucosyltransferase can be rapidly separated from the reaction system through magnetic separation technology. Through appropriate washing, it can continue to be used in new reaction systems. After 5 repeated uses, the reduction in enzyme activity can be maintained within 4% of the original activity.

Therefore, this invention can achieve reuse of Helicobacter pylori α-1,3-fucosyltransferase, increase target product yield, reduce production costs, and has continuity, suitable for continuous and automated production.

### BRIEF DESCRIPTION OF THE DRAWINGS

Figure 1: Sequence comparison of mutants of Helicobacter pylori α-1,3-fucosyltransferase;
Figure 2: SDS-PAGE and size exclusion chromatography (SEC) analysis of mutants of Helicobacter pylori α-1,3-fucosyltransferase;
Figure 3: Schematic diagram of GDP-Glo^{™} glycosyltransferase assay;
Figure 4: Schematic diagram of detecting glycosyltransferase activity by flow cytometry;
Figure 5: Comparison of enzyme activity between magnetic bead-coupled and free fucosyltransferase mutants by GDP-Glo^{™} glycosyltransferase assay;
Figure 6: Comparison of enzyme activity between magnetic bead-coupled and free fucosyltransferase mutants by flow cytometry detection;
Figure 7: Schematic diagram of repeated use of magnetic bead-coupled fucosyltransferase;
Figure 8: Activity detection of repeated use of magnetic bead-coupled fucosyltransferase.

### DETAILED DESCRIPTION

The following will further describe the present disclosure in conjunction with specific embodiments, and the advantages and features of the present disclosure will become clearer as described. However, these embodiments are only exemplary and do not constitute any limitation on the scope of protection defined by the claims of the present disclosure.

### Example 1. Preparation of Mutants of Helicobacter pylori α-1,3-Fucosyltransferase

### 1. Preparation of Mutants DNA

Site-directed mutagenesis is achieved by introducing base substitution mutations in the target gene during gene amplification by PCR DNA polymerase. In the present invention, the DNA sequence of the recombinant truncated form (FucTd) of Helicobacter pylori α-1,3-fucosyltransferase is shown as SEQ ID NO.2, and the translated amino acid sequence is shown as SEQ ID NO.1. The mutation strategy for Helicobacter pylori α-1,3-fucosyltransferase in the present invention is as follows:
FucTd-2C: Mutation of C-S at position 169and C-S at position 238 of SEQ ID NO.1, the mutated amino acid sequence is shown as SEQ ID NO.3, and the DNA sequence is described as SEQ ID NO.4.

FucTd_C406: C added at position 406 of SEQ ID NO.1; the mutated amino acid sequence is shown as SEQ ID NO.5, and the DNA sequence is described as SEQ ID NO.6.

FucTd_C412: C added at position 412 of SEQ ID NO.1; the mutated amino acid sequence is shown as SEQ ID NO.7, and the DNA sequence is described as SEQ ID NO.8.

FucTd-2C_C406: C-S at position 169 + C-S at position 238 + C added at position 406 of SEQ ID NO.1; the mutated amino acid sequence is shown as SEQ ID NO.9, and the DNA sequence is described as SEQ ID NO.10.

The above recombinant truncated form (FucTd) and mutants all carry a histidine tag at the carboxyl terminus for purification of recombinant proteins. FucTd has only two cysteines, all of which are replaced by serine in the present invention to obtain FucTd-2C. Secondly, based on FucTd, free cysteines are added by mutation, that is, a cysteine is inserted at the N-terminus (position 406) or C-terminus (position 412) of the histidine tag, respectively obtaining FucTd_C406 and FucTd_C412. Based on FucTd-2C, a cysteine is inserted at the N-terminus (position 406) of the histidine tag, obtaining FucTd-2C_C406.

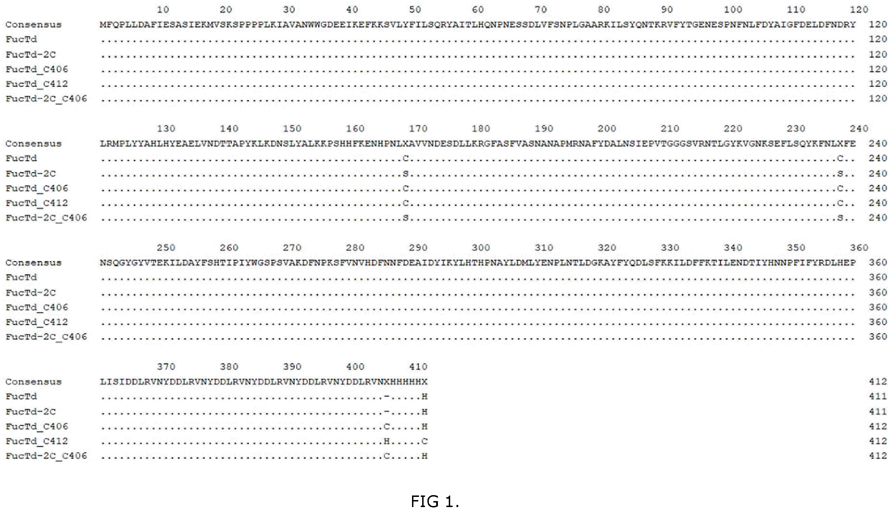

The sequence comparison diagram of the above four Helicobacter pylori α-1,3-fucosyltransferases is shown in Figure 1.

The DNA according to SEQ ID NO.2 was synthesized and cloned into pET41a by Suzhou Junji Biotechnology Co., Ltd. (MilliporeSigma^{™} pET-41a(+) DNA Vector, Catalog No.: 70-556-3). Suzhou Junji Biotechnology Co., Ltd. was commissioned to synthesize corresponding primers according to sequences SEQ ID NO.4, SEQ ID NO.6, SEQ ID NO.8, and SEQ ID NO.10:
FucTd-2C
   C169S Forward: 5'-CCGAACCTGTCCGCGGTGGTG-3'
   C169S Reverse: 5'-CACCACCGCGGACAGGTTCGG-3'
   C238S Forward: 5'-TTTAACCTGTCCTTTGAAAAC-3'
   C238S Reverse: 5'-GTTTTCAAAGGACAGGTTAAA-3'
FucTd_C406
   5'-CGTAAACTGCCATCATCACC-3'
   5'-GGTGATGATGGCAGTTTACG-3'
FucTd_C412
   5'-CCATCATCACTGCTAATAAAG-3'
   5'-CTTTATTAGCAGTGATGATGG-3'
FucTd-2C_C406
   Forward: 5'-CATATGTTTCAGCCGCTGCTG-3'
   Reverse: 5'-GTTTACGCGTAAGTCATCGTAATTG-3'
FucTd-2C, FucTd_C406, and FucTd_C412 were firstly prepared, using recombinant truncated form (FucTd) SEQ ID NO.1 as DNA template; when preparing FucTd-2C_C406, FucTd-2C SEQ ID NO.3 was used as DNA template.

PCR reaction conditions were as follows:
To a 50 µL PCR system, add 5 µL 10× TITANIUM Taq Buffer (400 mM Tricine-KOH (pH 8.0 at 25°C), 160 mM KCl, 35 mM MgCl₂, 37.5 µg/mL BSA), 1 µL 50× Diversify dNTP Mix (dATP, dCTP, dGTP, and dTTP each 10 mM), 10 µM each of two primers, 50 ng DNA template, 20 units TITANIUM Taq, and PCR-grade pure water to bring the final volume to 50 µL. The above reagents were from Clontech, Catalog No. 639141.

After thoroughly mixing and centrifuging the reaction components, the PCR tube was placed in a PCR thermal cycler. PCR reaction parameters were set as: 94°C for 30 seconds; 25 cycles (94°C for 30 seconds, 68°C for 1 minute); 68°C for 1 minute; 4°C hold.

The PCR products obtained above were subjected to agarose gel electrophoresis, gel extraction and purification, then according to a 10 µL volume reaction system: take 1 µL T-vector (50 ng) (T-vector is pMD^{™}19-T Vector Cloning Kit, Takara Cat. No. 6013), add equimolar amount of PCR product. Add 1 µL 10× Buffer containing ATP, appropriate units of T4 DNA ligase, and bring to 10 µL with ddH₂O. Ligate overnight at 16°C. The ligation product was transformed into E. coli, briefly described as follows:
(1) Preheat LB plates containing Amp, X-Gal, and IPTG at 37°C.
(2) Add 10 µL ligation product to 100 µL competent cells, and incubate on ice for 30 min.
(3) Transfer the centrifuge tube to a 42°C water bath, heat shock for 90 seconds, then without shaking the centrifuge tube, quickly place it on ice for 2 min.
(4) Add 300 µL SOC medium to the centrifuge tube, mix with pipette tip, and shake gently at 37°C, 150 rpm for 60 min.
(5) Spread 200 µL of the transformed bacterial solution evenly on LB plates containing 50 mg/mL Amp, 20 mg/mL X-gal, and 200 mg/mL IPTG, and incubate inverted overnight at 37°C. Select white colonies and commission Suzhou Junji Biotechnology Co., Ltd. for sequencing. According to the sequencing results, select correctly mutated sequences and clone them into pET41a (MilliporeSigma^{™} pET-41a(+) DNA Vector, Catalog No.: 70-556-3).

### 2. Expression of Mutants

The expression of mutants of Helicobacter pylori α-1,3-fucosyltransferase is briefly described as follows:
(1) Add 2 µL plasmid to 100 µL BL21(DE3) competent cells (ThermoFisher, Catalog No.: EC0114), mix immediately, and place on ice for 30 min.
(2) Heat shock at 42°C for 90 s, quickly ice bath for 2 min.
(3) Add 500 µL LB medium, and shake culture at 37°C, rpm ≤ 200 for 60 min.
(4) Centrifuge at 6000 rpm for 1 min, discard most of the supernatant, leaving about 100-150 µL, resuspend the bacteria, and spread on LB plates containing Amp, and culture overnight at 37°C.
(5) Small-scale expression: Pick one single colony into 1 mL LB medium with Amp resistance, shake culture at 37°C, 220 rpm for about 5 h, then add 2.5 mL LB liquid medium containing Amp to the previous tube, and shake culture overnight at 37°C, 220 rpm.
(6) Transfer the overnight cultured bacterial solution to 20 mL LB medium containing Amp at a 1:50 ratio, culture at 37°C, 220 rpm until OD₆₀₀ = 0.6 (about 3 h), add IPTG to a final concentration of 0.5 mM, and culture at 20°C, 220 rpm for 16 h.
(7) Measure the OD₆₀₀ of the culture, take 10 OD of bacterial solution, centrifuge at 10,000 rpm for 2 min, and remove the supernatant.
(8) Resuspend the bacteria in 1 mL lysis buffer (10 mM Tris-HCl, pH 8.0), and perform ultrasonic lysis on ice. Ultrasonic conditions: 130 W, 4 min, on 3 s, off 3 s.
(9) After ultrasonic treatment, centrifuge the lysate at 12,000 rpm for 10 min to obtain the supernatant (lysis supernatant); ultracentrifuge the supernatant at 125,000 g at 4°C.

The expression and purification yields of the recombinant truncated form (FucTd) of Helicobacter pylori α-1,3-fucosyltransferase and various mutants are shown in the purification yield column of Table 1.

**Table 1. Comparison of expression, purification and enzyme activity of mutants of Helicobacter pylori α-1,3-fucosyltransferase**

| Protein name | Induction expression conditions | Purification yield (mg/L medium) | Enzyme activity ratio (GDP-Glo Glycosyltransfe rase assay) | Enzyme activity ratio (Enzyme-mediated antibody-cell conjugation/FA CS) |
|---|---|---|---|---|
| FucTd | 0.2 mM IPTG, 20°C overnight | 10 | 1 | 1 |
| FucTd-2C | 0.2 mM IPTG, 20°C overnight | 12 | 1.3 | 0.92 |
| FucTd_C406 | 0.2 mM IPTG, 20°C overnight | 50 | 1.1 | 0.8 |
| FucTd_C412 | 0.2 mM IPTG, 20°C overnight | 30 | 1 | 0.84 |
| FucTd-2C_C406 | 0.2 mM IPTG, 20°C overnight | 8 | 1.4 | 0.96 |

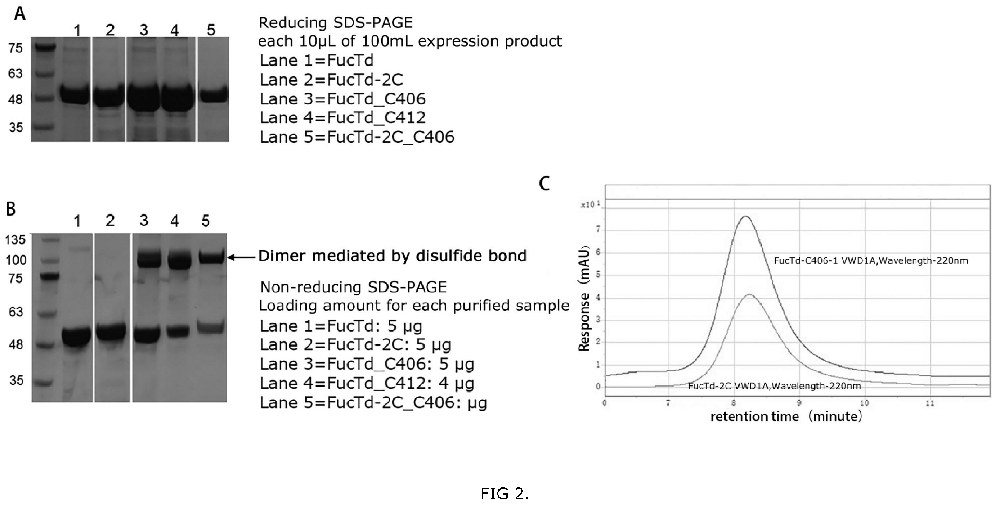

Under the same induced expression conditions, the expression and purification yields of the cysteine-removed mutants FucTd-2C and FucTd-2C_C406 did not change significantly compared to the recombinant truncated form (FucTd) (12 mg/L vs 8 mg/L vs 10 mg/L), indicating that the two cysteines (C169 and C238) in Helicobacter pylori α-1,3-fucosyltransferase do not participate in and maintain protein structure through disulfide bond formation. FucTd_C406 and FucTd_C412 also did not show multimers or reduced yield due to the insertion of additional cysteines, suggesting that C406 and C412 do not form disulfide bonds with C169 and C238, and C169 and C238 are likely not exposed on the protein molecule surface. Surprisingly, FucTd_C406 and FucTd_C412 not only did not show reduced yield, but instead increased yield by 3 to 5 times (50 mg/L vs 30 mg/L). It is speculated that Helicobacter pylori α-1,3-fucosyltransferase may form dimers to increase stability; the additionally added cysteines can further reinforce the formation of homodimers. SDS-PAGE and size exclusion chromatography (SEC) analysis also confirmed this. As shown in Figure 2, Panel A shows that on reducing SDS-PAGE, FucTd and all mutants appear as monomers; Panel B shows that on non-reducing SDS-PAGE, FucTd and FucTd-2C remain monomers, proving that the two cysteines at positions 169 and 238 are unrelated to dimer formation; while other mutants FucTd_C406, FucTd_C412, and FucTd-2C_C406 all can form dimers, and dimers are mediated by disulfide bonds and other factors, indicating that the cysteines inserted at positions 406 and 412 are both related to dimer formation. Panel C compares the molecular size of FucTd-2C and FucTd_C406 by size exclusion chromatography (SEC); both show single peaks with similar column retention times, with molecular weights around 100 kD. This indicates that although they differ on SDS-PAGE, both will form dimers under non-denaturing native conditions.

### 3. Enzyme Protein Purification

According to the above expression induction conditions, the culture volume was expanded to 100 mL, and the supernatant of the ultrasonic lysate was obtained. According to the manual, the supernatant was applied to a HiTrap Chelating HP column and eluted with 20 mM imidazole solution. The eluate was pooled and the solvent was exchanged to 50 mM Tris buffer (pH 8.0) by dialysis, then further purified by gel filtration chromatography (Superdex 200, GE Healthcare) to obtain proteins with high purity, producing proteins with more than 98% homogeneity. Using bovine serum albumin as a benchmark, protein concentration was determined using a Bio-Rad protein assay kit based on the Bradford method.

### Example 2. Enzyme Activity Analysis of Recombinant Truncated Form and Mutants of Helicobacter pylori α-1,3-Fucosyltransferase by GDP-Glo^{™} Glycosyltransferase Assay

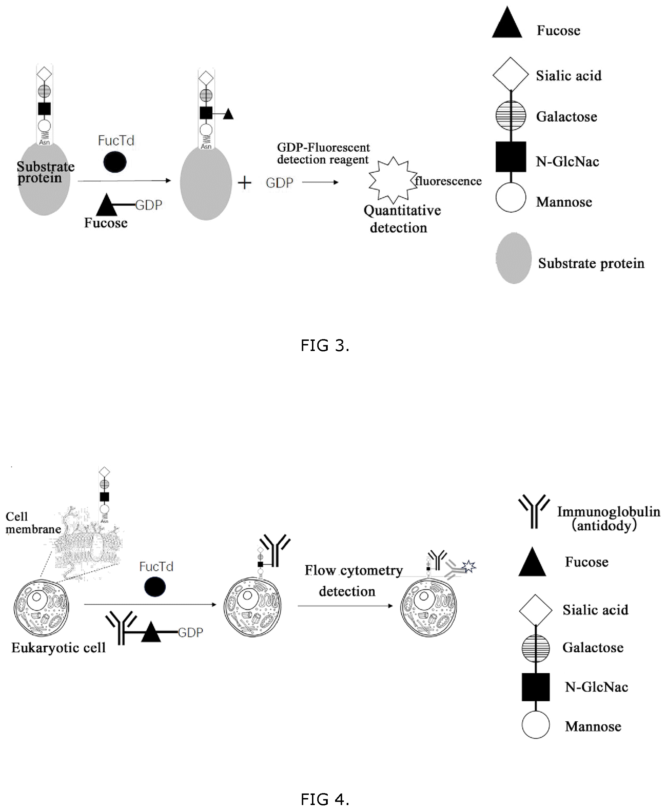

The enzyme activity of the recombinant truncated form and mutants of Helicobacter pylori α-1,3-fucosyltransferase was detected using a Promega kit (principle as shown in Figure 3), using high-purity GDP-fucose as the donor substrate and fetuin as the receptor substrate. Enzyme activity determination was performed according to the manual (Promega GDP-Glo^{™} Glycosyltransferase Assay). 100 µM ultra-pure GDP-fucose (Promega Cat. #VA1097) was used as donor substrate, and 40 µM fetuin (Promega Cat. #V4961) was used as receptor substrate. Serially diluted purified fucosyltransferase mutants (0 ng, 5 ng, 10 ng, 20 ng, 30 ng, 40 ng, 50 ng, 60 ng, 70 ng, 80 ng, 90 ng, 100 ng) were added sequentially. All enzyme reactions were carried out in white 96-well plates in a volume of 25 µL. Incubate at room temperature for 60 minutes, place in GloMax^{®} 96 Microplate Luminometer (Cat. #E6501), read at room temperature, and plot with enzyme mass in the reaction system as the x-coordinate and data read on the luminometer as the y-coordinate, using slope as the activity index. The results are summarized in the enzyme activity ratio (GDP-Glo glycosyltransferase assay) column of Table 1. There were no significant differences in enzyme activity between each mutant and the recombinant truncated form (FucTd), suggesting that the wild-type C169 and C238 in the sequence and the additionally introduced C406 and C412 do not directly participate in the formation of the enzyme active center.

### Example 3. Comparison of Enzyme Activity of Recombinant Truncated Form and Mutants of Helicobacter pylori α-1,3-Fucosyltransferase by Flow Cytometry Detection

The principle is shown in Figure 4. Eukaryotic cell membranes have abundant glycoprotein molecules that can serve as receptor substrates for fucosyltransferase. In the present invention, NK-92 cells are used as receptor substrates. Trastuzumab labeled with GDP-fucose is used as the donor substrate. The mean fluorescence intensity (MFI) of flow cytometry can compare the amount of target molecules on the cell membrane under the same conditions and can serve as an indicator of enzyme reaction products.

NK-92 (natural killer cells from a patient with human malignant non-Hodgkin's lymphoma) was purchased from Wuhan Procell Life Science & Technology Co., Ltd. The preparation of trastuzumab and the experimental process of labeling trastuzumab with GDP-fucose are described in Example 4. To NK-92 cells at 5×10⁶/mL, 100 µg/mL recombinant truncated form or mutants of Helicobacter pylori α-1,3-fucosyltransferase, and 150 µg/mL GDP-fucose-labeled trastuzumab were added respectively, and incubated at room temperature for 30 minutes. Wash with PBS pH 7.4, centrifuge at 500×g, and remove the supernatant. Resuspend NK-92 cells in PBS pH 7.4, co-incubate with anti-hIgG-Biotin antibody (Beijing Baiaolaibo Technology Co., Ltd., Product No. F030822, 1:200 dilution) at 4°C for 30 minutes. After one times of PBS wash, stain with Streptavidin-PE (BioLegend, Inc., Product No. 405204, 1:200 dilution), co-incubate at 4°C for 30 minutes. After one times of PBS wash, detect with flow cytometer (Beijing Cenglang Biotechnology Co., Ltd., Product Model MateCyte) and analyze with NovoExpress software. Under the same experimental conditions, mean fluorescence intensity (MFI) can reflect the amount of target protein on the cell membrane surface. Comparing the mean fluorescence intensity (MFI) of NK-92 cells treated with the recombinant truncated form and mutants of Helicobacter pylori α-1,3-fucosyltransferase, as summarized in the enzyme activity ratio (enzyme-mediated antibody-cell coupling/FACS) column of Table 1, there was no significant difference compared to the recombinant truncated form.

### Example 4. Preparation of GDP-Fucose-Labeled Trastuzumab

Trastuzumab (trastuzumab for injection), according to the sequence published in the literature, corresponding DNA was designed and plasmid DNA was synthesized by Suzhou Junji Biotechnology Co., Ltd., and cloned into vector pRM293 (pRM293 was modified based on plasmid pTT5, specifically see Shi C. Purification and characterization of a recombinant G-protein-coupled receptor, Saccharomyces cerevisiae Ste2p, transiently expressed in HEK293 EBNA1 cells. Biochemistry. 2005;44(48):15705-15714.). Suzhou Junji Biotechnology Co., Ltd. was commissioned to transiently transfect the plasmid into HEK293 cells (National Research Council, Canada), and affinity purification was performed using MabSelect Sure (Protein A, GE Healthcare).

GDP-fucose labeling of trastuzumab is divided into the following two steps:
1. NHS ester-activated crosslinkers and the compound to be labeled react with primary amines on trastuzumab under physiological to weakly alkaline conditions (pH 7.2 to 9) to form stable amide bonds. Using this principle, the NHS on TCO-PEG4-NHS reacts with primary amines on trastuzumab protein to obtain TCO-PEG4-trastuzumab. The specific method and process are as follows:

React 400 µg purified trastuzumab protein with 150 µg TCO-PEG4-NHS (Shanghai Pairui Pharmaceutical Technology Co., Ltd., Product No. A34125), and add 20 mM HEPES buffer (pH 7.0~7.5, Thermo Fisher Scientific (China) Co., Ltd., Product No. 15630), incubate at room temperature for 30 minutes, then add 5 µmol Tris buffer to terminate the reaction, incubate at room temperature for 5 minutes. Add the reaction product to a PD SpinTrap G-25 desalting column (Cytiva, Product No. 28918004), centrifuge at 800×g, remove unreacted and small molecules generated by the reaction, and obtain pure TCO-PEG4-trastuzumab.

2. Based on the inverse demand Diels-Alder cycloaddition reaction between trans-cyclooctene and tetrazine reaction pairs, the linkage between TCO (trans-cyclooctene) and Tz (tetrazine) forms a dihydropyridazine bond. Using this principle, TCO-PEG4-trastuzumab reacts with GDP-fucose derivative GDP-Fucose-Triazole-PEG4-Tz to obtain GDP-Fucose-Triazole-PEG4-PEG4-trastuzumab, i.e., GDP-fucose-labeled trastuzumab. Specifically:

The TCO-PEG4-trastuzumab obtained in the previous step is further reacted with 30 µg GDP-Fucose-Triazole-PEG4-Tz (synthesized by Yantang Biotechnology Co., Ltd., Product No. YT-HJP-3-29), incubated at room temperature for 30 minutes, then the reaction product is added to a PD SpinTrap G-25 desalting column (Cytiva, Product No. 28918004), centrifuged at 800×g, remove unreacted and small molecules generated by the reaction, and obtain pure GDP-fucose-labeled trastuzumab.

According to MALDI-TOF detection, the labeled trastuzumab carries about 6 to 10 GDP-fucose derivatives. Hereafter, unless specifically stated, trastuzumab refers to GDP-fucose-coupled trastuzumab.

### Example 5. Magnetic Bead Coupling of Fucosyltransferase

The mutants of Helicobacter pylori α-1,3-fucosyltransferase FucTd_C406, FucTd_C412, and FucTd-2C_C406 all have an additionally introduced free cysteine, which is coupled to the surface of magnetic beads through site-specific coupling technology targeting cysteine. Since FucTd-2C has no free Cys, it does not have Cys-mediated coupling ability, and FucTd-2C was not selected for coupling experiments in this example. The methods include two types:
1. A stable thioether bond is formed between the thiol group of cysteine and biotin through maleimide (NHS), and then the mutant is coupled to streptavidin-coated magnetic beads using the high affinity between biotin and streptavidin. The method is briefly described as follows: Dissolve FucTd_C406, FucTd_C412, and FucTd-2C_C406 proteins in PBS buffer pH 7.4, and prepare a final concentration of 2 mg/mL. Freshly prepare BMCC-biotin (1-biotinamido-4-4'-[maleimidomethyl]cyclohexanecarboxamido-butane) (ThermoFisher, Cat. No.: 21900), add 500 µL dimethyl sulfoxide to 2.1 mg BMCC-biotin to prepare an 8 mM stock solution. Add 17 µL BMCC-biotin to 1 mL protein solution. React at room temperature for 2 hours. Add the reaction product to a PD SpinTrap G-25 desalting column (Cytiva, Product No. 28918004), centrifuge at 800×g, remove unreacted and small molecules generated by the reaction, and obtain biotin-labeled protein. Detect biotinylation level using Pierce^{™} Fluorescence Biotin Quantitation Kit (ThermoFisher, Cat. No.: 46610), showing similar biotinylation levels for the three proteins. Mix 10 µg biotinylated protein with 100 µL streptavidin Dynabeads^{™} M-280 (ThermoFisher, Cat. No.: 11205D) in PBS buffer at room temperature, co-incubate for 30 minutes; magnetically isolate the bead-coupled proteins, wash them 4-5 times with 0.1% BSA-PBS, and set aside for later use.
2. Utilize the free cysteine of the mutant to directly couple to the epoxy group on epoxy magnetic beads, forming a covalent bond. The method is briefly described as follows: Dynabeads^{™} M-270 Epoxy magnetic beads (ThermoFisher, Cat. No.: 14301) are monodisperse 2.8-µm superparamagnetic polystyrene beads coated with a hydrophilic polyether cross-linked layer. The Epoxy functional groups of the magnetic beads form disulfide bonds with free cysteines of proteins through thiol groups. The coupling method is briefly described as follows: Weigh 5 mg lyophilized magnetic beads (3.3×10⁸), wash and resuspend in 100 µL PBS (pH 7.4); add 100 µL target protein, mix thoroughly, then add 100 µL 3 M ammonium sulfate. Mix repeatedly and co-incubate at 37°C for 16-24 hours, then wash 4 times with PBS (pH 7.4), and resuspend the magnetic beads in 165 µL PBS (pH 7.4) for standby use.

### Example 6. Comparison of Enzyme Activity Between Magnetic Bead-Coupled and Free Fucosyltransferase by GDP-Glo^{™} Glycosyltransferase Assay

Using bovine serum albumin as a benchmark, determine protein concentration using a Bio-Rad protein assay kit based on the Bradford method, and adjust free protein and magnetic bead-coupled protein to 50 µg/mL. 100 µM ultra-pure GDP-fucose (Promega Cat. #VA1097) is used as donor substrate, and 40 µM fetuin (Promega Cat. #V4961) is used as receptor substrate. To 25 µL reaction system, add fucosyltransferase mutants and their magnetic bead coupl respectively, serially diluted to 0 ng, 5 ng, 10 ng, 20 ng, 30 ng, 40 ng, 50 ng, 60 ng, 70 ng, 80 ng, 90 ng, 100 ng. All enzyme reactions are carried out in white 96-well plates. Incubate at room temperature for 60 minutes, place in GloMax^{®} 96 Microplate Luminometer (Cat. #E6501), read at room temperature, and plot with enzyme mass in the reaction system as the x-coordinate and data read on the luminometer as the y-coordinate, using slope as the activity index.

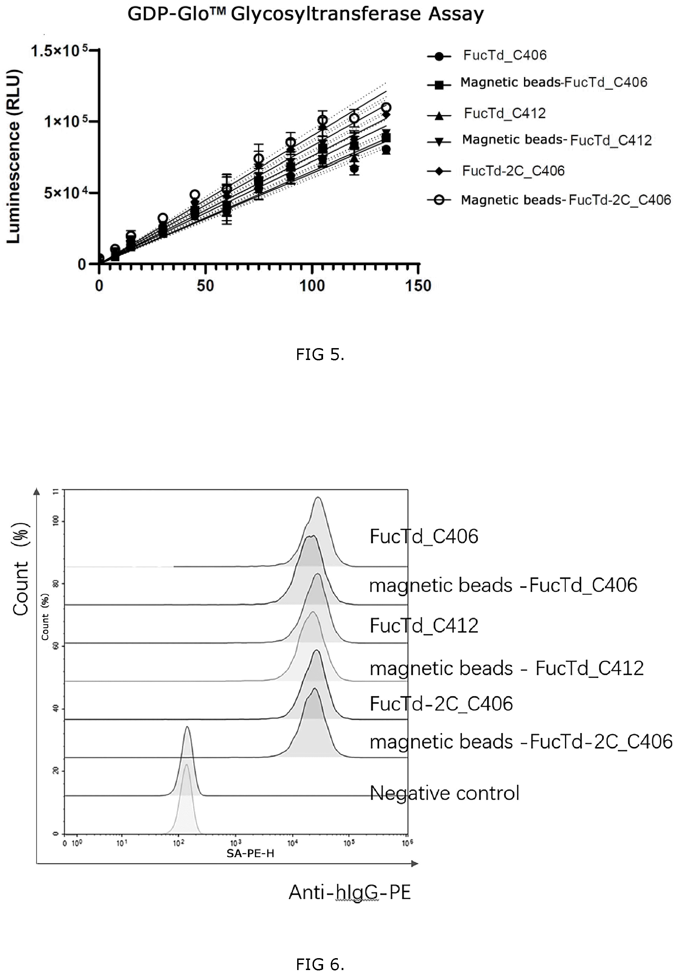

As shown in Figure 5 and Table 2, comparing the enzyme activity of the three mutants FucTd_C406, FucTd_C412, and FucTd-2C_C406 and their magnetic bead couples, magnetic bead coupling did not affect enzyme activity, and showed a slightly higher trend than the corresponding free protein enzyme activity, suggesting that the solid-phase surface can improve enzyme stability.

**Table 2. Comparison of enzyme activity of three mutants and their magnetic bead couples [108]**

| FucTd_C406 | magnetic bead-FucTd_C406 | FucTd_C412 | magnetic bead-FucTd_C412 | FucTd-2C_C406 | magnetic bead-FucTd-2C_C406 |
|---|---|---|---|---|---|
| Y=651.1*X | Y=756.7*X | Y=637.7*X | Y=718.3*X | Y=826.8*X | Y=898.7*X |
| Solid-phase immobilization increases enzymatic activity by 16% | | Solid-phase immobilization increases enzymatic activity by 13% | | Solid-phase immobilization increases enzymatic activity by 9**%** | |

### Example 7. Comparison of Enzyme Activity Between Magnetic Bead-Coupled and Free Fucosyltransferase Mutants by Flow Cytometry Detection

The mean fluorescence intensity (MFI) of flow cytometry can compare the amount of target molecules on the cell membrane under the same conditions and can serve as an indicator of enzyme reaction products. Similar to Example 3, NK-92 cells are used as receptor substrates, and trastuzumab labeled with GDP-fucose is used as the donor substrate. To NK-92 cells at 5×10⁶/mL, add Helicobacter pylori α-1,3-fucosyltransferase mutants (FucTd_C406, FucTd_C412, and FucTd-2C_C406) with final concentration of 50 µg/mL or their magnetic bead couples, and GDP-fucose-labeled trastuzumab with final concentration of 100 µg/mL, incubate at room temperature for 30 minutes. Wash with PBS pH 7.4, centrifuge at 500×g, and remove the supernatant. Resuspend NK-92 cells in PBS pH 7.4, co-incubate with anti-hIgG-Biotin antibody (Beijing Baiaolaibo Technology Co., Ltd., Product No. F030822, 1:200 dilution) at 4°C for 30 minutes. After one times PBS wash, stain with Streptavidin-PE (BioLegend, Inc., Product No. 405204, 1:200 dilution), co-incubate at 4°C for 30 minutes. After one times PBS wash, detect with flow cytometer (Beijing Cenglang Biotechnology Co., Ltd., Product Model MateCyte) and analyze with NovoExpress software. Under the same experimental conditions, mean fluorescence intensity (MFI) can reflect the amount of target protein on the cell membrane surface. As shown in Figure 6, Helicobacter pylori α-1,3-fucosyltransferase mutants (FucTd_C406, FucTd_C412, and FucTd-2C_C406) or their magnetic bead couples can all complete the transfer of GDP-fucose-labeled trastuzumab to NK-92 cells. There is no significant difference between free enzyme and magnetic bead-coupled enzyme; coupling efficiency is high, and 100% coupling can be achieved at room temperature in 30 minutes; MFI can indirectly reflect the amount of target protein on the cell membrane surface. Table 3 shows that magnetic bead-coupled FucTd_C412 and FucTd-2C_C406 can both increase coupling amount, while magnetic bead-coupled FucTd_C406 does not. Combined with the GDP-Glo^{™} experiment in Example 6, only FucTd_C406 showed slightly reduced enzyme coupling amount compared to free enzyme due to solid-phase coupling, while others showed increased enzyme activity or coupling amount due to coupling with the solid phase.

**Table 3. Comparison of coupling amount of three mutants and their magnetic bead couples using mammalian cells as substrates**

| | FucTd_C4 06 | magnetic bead-FucTd_C4 06 | FucTd_C4 12 | magnetic bead-FucTd_C4 12 | FucTd-2C_C406 | magnetic bead-FucTd-2C_C406 |
|---|---|---|---|---|---|---|
| MFI | 29240 | 23426 | 24562 | 28006 | 25298 | 26983 |
| | Solid-phase immobilization reduces enzymatic activity by 20% | | Solid-phase immobilization increases enzymatic activity by 14% | | Solid-phase immobilization increases enzymatic activity by 6% | |

### Example 8. Activity Detection of Reusable Magnetic Bead-Coupled Fucosyltransferase Mutants

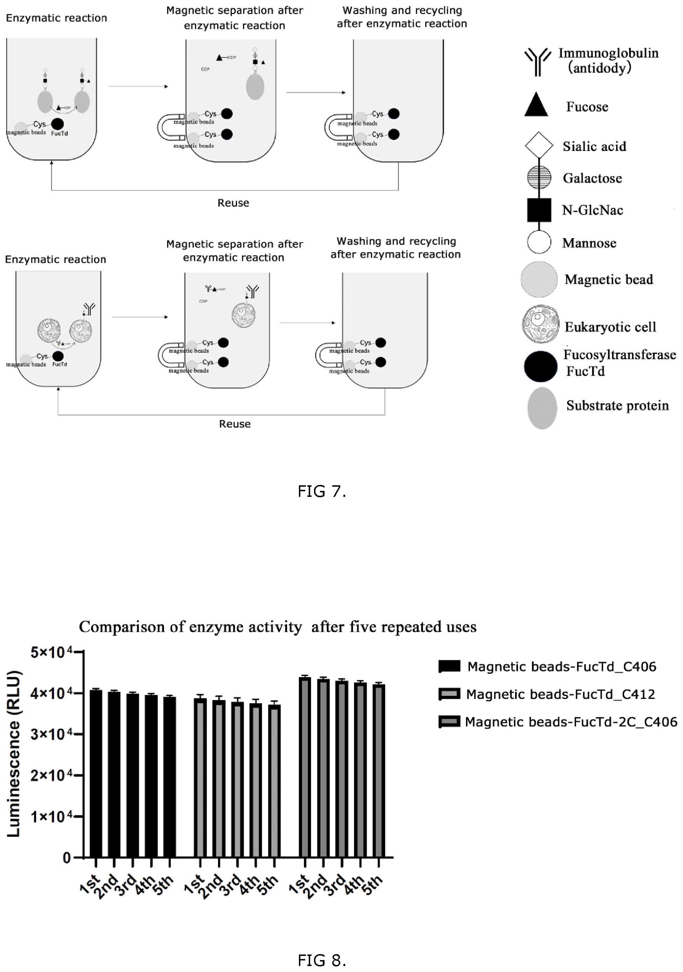

As shown in Figure 7, Panel A shows receptor substrates as specific protein molecules, and Panel B shows receptor substrates as mammalian cells; both different substrates can reuse magnetic bead-coupled fucosyltransferase to achieve coupling modification of specific protein molecules or mammalian cells. The enzyme is coupled to magnetic beads through site-specific coupling, and the catalytic reaction is completed in solution, then through magnetic separation, the magnetic beads are separated and washed from the reactants, convenient for recovery and reusable. To detect the stability of the enzyme during repeated use, the following experiment was implemented:

Using bovine serum albumin as a benchmark, determine protein concentration using a Bio-Rad protein assay kit based on the Bradford method, and adjust magnetic bead-coupled FucTd_C406, FucTd_C412, and FucTd-2C_C406 to 50 µg/mL. 100 µM ultra-pure GDP-fucose (Promega Cat. #VA1097) is used as donor substrate, and 40 µM fetuin (Promega Cat. #V4961) is used as receptor substrate. To 100 µL reaction system, add 100 ng of magnetic bead couples of three fucosyltransferase mutants respectively, incubate at room temperature for 60 minutes, place the centrifuge tube in a DynaMag^{™}-2 magnetic bead separation rack, let stand for magnetic attraction for 2 minutes, transfer the supernatant to a white 96-well plate, place in GloMax^{®} 96 Microplate Luminometer (Cat. #E6501), read data at room temperature as the data for the first use of magnetic beads. Then wash the magnetic beads 3 times on the magnetic rack, remove the supernatant, add fresh donor substrate and receptor substrate, incubate at room temperature for 60 minutes, measure the reading of the supernatant as described above as the data for the second use of magnetic beads. Proceed to the third, fourth, and fifth times in sequence.

As shown in Figure 8, after five repeated uses, the reduction in enzyme activity is within 4% (as shown in Table 4). After each wash, there will be a small loss of magnetic beads, which may be the main reason for the gradual decrease in enzyme activity.

**Table 4. Comparison of enzyme activity of magnetic bead couples of three mutants after five repeated uses.**

| number of reuse cycles | magnetic bead-FucTd_C406 | magnetic bead-FucTd_C412 | magnetic bead-FucTd-2C_C406 |
|---|---|---|---|
| | | | |
| 1 | 40612±436 | 38913±978 | 43806±524 |
| 2 | 40306±432 | 38326±968 | 43417±518 |
| 3 | 39803±428 | 37643±958 | 42883±513 |
| 4 | 39504±423 | 37563±949 | 42553±508 |
| 5 | 39109±419 | 37188±939 | 42127±503 |
| activity reduction rate after 5 reuse cycles | 3.70% | 4% | 3.80% |

### Industrial practicality

The present invention discloses a mutant of Helicobacter pylori α-1,3-fucosyltransferase, which can be industrially produced and has industrial utility.

## Claims

1. A mutant of Helicobacter pylori α-1,3-fucosyltransferase, **characterized in that** the amino acid sequence of said mutant of Helicobacter pylori α-1,3-fucosyltransferase is shown as SEQ ID NO.3, or shown as SEQ ID NO.5, or shown as SEQ ID NO.7, or shown as SEQ ID NO.9.

2. A polynucleotide encoding the mutant of Helicobacter pylori α-1,3-fucosyltransferase according to claim 1.

3. The polynucleotide according to claim 2, **characterized in that**:
the polynucleotide sequence encoding the mutant of Helicobacter pylori α-1,3-fucosyltransferase having the amino acid sequence shown as SEQ ID NO.3 is shown as SEQ ID NO.4; or
the polynucleotide sequence encoding the mutant of Helicobacter pylori α-1,3-fucosyltransferase having the amino acid sequence shown as SEQ ID NO.5 is shown as SEQ ID NO.6; or
the polynucleotide sequence encoding the mutant of Helicobacter pylori α-1,3-fucosyltransferase having the amino acid sequence shown as SEQ ID NO.7 is shown as SEQ ID NO.8;or the polynucleotide sequence encoding the mutant of Helicobacter pylori α-1,3-fucosyltransferase having the amino acid sequence shown as SEQ ID NO.9 is shown as SEQ ID NO.10.

4. An expression vector expressing the mutant of Helicobacter pylori α-1,3-fucosyltransferase according to claim 1, **characterized in that** said expression vector contains the polynucleotide according to claim 2 or 3.

5. A host cell expressing the mutant of Helicobacter pylori α-1,3-fucosyltransferase according to claim 1, **characterized in that** said host cell contains the expression vector according to claim 4.

6. A mutant of Helicobacter pylori α-1,3-fucosyltransferase according to claim 1 coupled with a solid phase carrier.

7. The mutant of Helicobacter pylori α-1,3-fucosyltransferase coupled with a solid phase carrier according to claim 6, **characterized in that** said solid phase carrier is magnetic beads.

8. A method for preparing the mutant of Helicobacter pylori α-1,3-fucosyltransferase coupled with magnetic beads according to claim 7, **characterized in that** said method comprises:
(1) directly coupling free thiol groups on the mutant of fucosyltransferase to the surface of magnetic beads, or
(2) performing site-specific biotinylation of free thiol groups, and then coupling to magnetic beads by means of biotin-streptavidin affinity.

9. A method for repeatedly applying the mutant of Helicobacter pylori α-1,3-fucosyltransferase coupled with a solid phase carrier according to claim 6 to transfer a donor substrate coupled with a biomacromolecule to a receptor substrate, **characterized in that** said method comprises the following steps:
after completing the transfer of GDP-fucose coupled with a biomacromolecule to target cells, washing the magnetic beads in a magnetic field environment, removing the supernatant, adding fresh donor substrate and receptor substrate, and again transferring the donor substrate coupled with a biomacromolecule to the receptor substrate.

10. The method according to claim 9, **characterized in that** said biomacromolecule is an antibody.
